# EUROPEAN PATENT APPLICATION

(11) **EP 4 570 297 A1**
(43) Date of publication of application: **18.06.2025**
(21) Application number: 23908956.8
(22) Date of filing: 12.05.2023
(51) Int. Cl.: A61M 25/10, A61B 17/22

(54) **EXTENSION CATHETER AND MEDICAL INSTRUMENT**

(30) Priority: 29.12.2022 CN 202223593958 U
(71) Applicant: Brosmed Medical Co., Ltd., Dongguan, Guangdong 523808 (CN)
(72) Inventor: ZHANG, Zhijun, Dongguan, Guangdong 523808 (CN); LIU, Chaosheng, Dongguan, Guangdong 523808 (CN); ZHONG, Guansheng, Dongguan, Guangdong 523808 (CN)
(74) Representative: Meyer, Thorsten
(86) International application number: PCT/CN2023/093875
(87) International publication number: WO 2024/139008

(57) **Abstract**

An extension catheter and a medical instrument. The extension catheter comprises a catheter body (1), a push rod (2) and a balloon (3), the catheter body (1) having provided therein an extension channel (11), and the proximal end of the catheter body (1) being provided with a lead-in port (12); the push rod (2) is connected to the proximal end of the catheter body (1), and the push rod (2) has provided therein a filling channel (21); the balloon (3) is provided outside the proximal end of the catheter body (1), and the balloon (3) is in communication with the filling channel (21). The medical instrument comprises a guide catheter (6) and the extension catheter, the guide catheter (6) having provided therein a guide channel (61), the proximal end of the catheter body (1) extending into the distal end in the guide channel (61), and the balloon (3) abutting against the inner wall of the guide channel (61) after expansion. The extension catheter does not easily slide or slip in the guide catheter (6), and a contrast agent/drug may flow to the extension channel along the guide channel (61), to perform ultra-selective radiography/drug delivery; a suction device may be externally connected to achieve thrombus or plaque extraction functionality.

## Description

### CROSS REFERENCE TO RELATED APPLICATIONS

The present application claims priority to Chinese Patent Application No. 202223593958.5, filed on December 29, 2022, and entitled "Extension Catheter and Medical Device", which is hereby incorporated by reference in its entirety.

### FIELD OF THE INVENTION

The present application belongs to the field of medical devices, and particularly relates to an extension catheter and a medical device.

### BACKGROUND OF THE INVENTION

Percutaneous transluminal coronary intervention (PCI) is a technique that uses percutaneous puncture to insert balloon catheters or other related devices to relieve coronary artery stenosis or obstruction and restore coronary blood flow. With the continuous development of medical science, interventional medical surgery has also been significantly advanced. The existing extension catheter, when placed inside the guide catheter, may experience slippage or even dislodgement due to gaps in the fit.

### SUMMARY OF THE INVENTION

The objectives of the embodiments of the present application are to provide an extension catheter and a medical device to address the technical issues of existing extension catheters easily slipping or dislodging within the guide catheter.

In order to achieve the above objectives, the present application provides a technical solution as follows. An extension catheter includes:
a catheter body, an interior of the catheter body being provided with an extension channel for conveying a medical device, and a proximal end of the catheter body being provided with a guiding port;
a push rod, connected to the proximal end of the catheter body and forming a connecting portion, and an interior of the push rod being provided with an inflation channel; and
a balloon, provided on the catheter body and located at the proximal end of the catheter body, and an interior of the balloon being in communication with the inflation channel.

In one embodiment, the catheter body includes an inner layer, a reinforcement layer, and an outer layer arranged from inside to outside, and the extension channel is located in the inner layer.

In one embodiment, the extension catheter further includes a connecting member that is connected with the reinforcement layer and the push rod.

In one embodiment, the reinforcement layer includes a stainless steel braid or a spring.

In one embodiment, a material of the inner layer is polytetrafluoroethylene or linear low-density polyethylene.

In one embodiment, a material of the outer layer is selected from one or more of polyether-block-polyamide, nylon, and polyurethane elastomer.

In one embodiment, a hardness of a material of the outer layer decreases progressively from the proximal end to the distal end of the catheter.

In one embodiment, a material of the balloon is selected from one or more of silicone rubber, polyurethane elastomer, and thermoplastic elastomer.

In one embodiment, a cross-sectional shape of the push rod is elliptical, circular, or semi-circular.

In one embodiment, a material of the push rod is hypotube.

In one embodiment, the connecting member includes a connecting plate and multiple connecting strips connected to opposite sides of the connecting plate, the connecting plate and each of the connecting strips are connected to the reinforcement layer, the push rod is connected to the connecting plate, and each of the connecting strips wraps around and connects to the inner layer.

In one embodiment, a distal end of the push rod is provided with a first through-hole in communication with the inflation channel, and the outer layer is provided with a second through-hole corresponding to a position of the first through-hole, and the inflation channel is in communication with the interior of the balloon through the first and second through-holes.

In one embodiment, the guiding port is arranged in a beveled manner.

In one embodiment, the extension catheter further includes a catheter hub connected to a proximal end of the push rod, the catheter hub being provided with a third through-hole in communication with the inflation channel.

In one embodiment, a distal end of the catheter body is provided with a first radiopaque ring, and the proximal end of the catheter body is provided with a second radiopaque ring located inside the balloon.

In one embodiment, the balloon is vacuum-sealed and flatly adhered to the catheter body; the distal end of the balloon is closely welded to the outer layer of the catheter body to form a sealed end, while the proximal end of the balloon is closely welded to the outer layer and the distal end of the push rod.

The present application further provides a medical device including:
a guide catheter, an interior of the guide catheter being provided with a guide channel; and
the extension catheter, the proximal end of the catheter body being inserted into the guide channel, and the balloon, when expanded, abutting against an inner wall of the guide channel.

In one embodiment, the medical device further includes an aspirator connected to a proximal end of the guide catheter, the aspirator being in communication with the guide channel and used for aspirating thrombi.

The beneficial effects of the extension catheter provided by the embodiments of the present application are as follows. Compared with the existing technology, the extension catheter of the present application has a balloon provided at the proximal end of the catheter body and an inflation channel in the push rod that communicates with the balloon. The extension catheter is inserted along the proximal end of the guide catheter, with one end of the push rod protruding from the proximal end of the guide catheter and the distal end of the catheter body protruding from the distal end of the guide catheter. The proximal end of the catheter body is located at the distal end of the guide catheter. The balloon can be inflated through the inflation channel. After expansion, the outer wall of the balloon is tightly adhered to the inner cavity of the guide catheter, which prevents the extension catheter from slipping or dislodging within the guide catheter and enhances the axial stability of the extension catheter within the guide catheter.

The beneficial effects of the medical device provided by the embodiments of the present application are as follows. Compared with the existing technology, the medical device of the present application utilizes the aforementioned extension catheter. The proximal end of the catheter body is located at the distal end of the guide catheter. After the balloon is expanded, its outer wall is tightly adhered to the inner cavity of the guide catheter, completely sealing the inner cavity of the guide catheter. When the contrast agent or medication is introduced from the proximal end of the guide catheter, it flows along the guide channel and enters the extension channel through the guiding port for superselective angiography (displaying the pathological conditions of the coronary arteries by injecting the contrast agent) or drug delivery. The extension channel is in communication with the guide channel. The proximal end of the guide catheter can be connected to an aspirator, which can be used to aspirate thrombi and plaques by connecting the extension channel and the guide channel. In such a way, it avoids the inconvenience of frequently replacing interventional catheters during surgery, reduces the surgical time, lowers the surgical difficulty, and minimizes the patient's discomfort.

### BRIEF DESCRIPTION OF THE DRAWINGS

To more clearly illustrate the technical solutions in the embodiments of the present application, the following is a brief introduction to the drawings that will be used in the description of the embodiments or the existing technology. It is evident that the drawings described below are merely some embodiments of the present application. For those of ordinary skill in the art, other drawings can also be obtained based on these drawings without involving any inventive effort.
FIG. 1 is a structural diagram of the extension catheter provided by the embodiments of the present application.
FIG. 2 is a structural diagram of the connection between the push rod and the catheter body of the extension catheter provided by the embodiments of the present application;
FIG. 3 is a cross-sectional view of the catheter body of the extension catheter provided by the embodiments of the present application;
FIG. 4 is a cross-sectional view of the push rod of the extension catheter provided by the embodiments of the present application;
FIG. 5 is a structural diagram of the connecting member of the extension catheter provided by the embodiments of the present application;
FIG. 6 is a structural diagram of the connection between the catheter body and the guide catheter in the medical device provided by the embodiments of the present application.

Reference numerals:
1: Catheter body; 11: Extension channel; 12: Guiding port; 13: Inner layer; 14: Reinforcement layer; 15: Outer layer; 151: First radiopaque ring; 152: Second radiopaque ring;
2: Push rod; 21: Inflation channel; 22: First through-hole;
3: Balloon;
4: Catheter hub;
5: Connecting member; 51: Connecting plate; 52: Connecting strip;
6: Guide catheter; 61: Guide channel.

### DETAILED DESCRIPTION OF ILLUSTRATED EMBODIMENTS

To make the technical problems, technical solutions, and beneficial effects of the present application clearer, the following is a detailed description of the present application in conjunction with the drawings and embodiments. It should be understood that the specific embodiments described here are only for explaining the present application and do not limit the present application.

It should be noted that when an element is referred to as being "fixed on" or "provided on" another element, it can be directly or indirectly arranged on that element. When an element is referred to as being "connected to" another element, it can be directly or indirectly connected to that element.

It should be understood that terms such as "length", "width", "up", "down", "front", "back", "left", "right", "vertical", "horizontal", "top", "bottom", "inner", "outer", etc., indicate directions or positional relationships based on the orientation shown in the figures. These terms are used only for convenience in describing the present application and simplifying the description, not to indicate or imply that the device or element must have a specific orientation, construction, or operation. Therefore, these terms should not be construed as limitations of the present application.

In addition, terms such as "first" and "second" are used for descriptive purposes only and should not be interpreted as indicating or implying relative importance or the number of technical features indicated. Thus, features defined by "first" and "second" may explicitly or implicitly include one or more of such features. In the description of the present application, "multiple" means two or more, unless otherwise specifically defined.

The phrases referring to "one embodiment", "some embodiments", or "embodiments" in the specification of the present application mean that one or more embodiments of the present application includes the specific features, structures, or characteristics described in conjunction with these embodiments. Therefore, the phrases "in one embodiment", "in some embodiments", "in other embodiments", and "in additional embodiments" appearing at different places in this specification do not necessarily refer to the same embodiment, but mean "one or more but not all embodiments," unless otherwise specifically emphasized. Moreover, specific features, structures, or characteristics may be combined in any suitable manner in one or more embodiments.

The term "proximal" generally refers to the end of a component closer to the operator, while the term "distal" refers to the end farther from the operator.

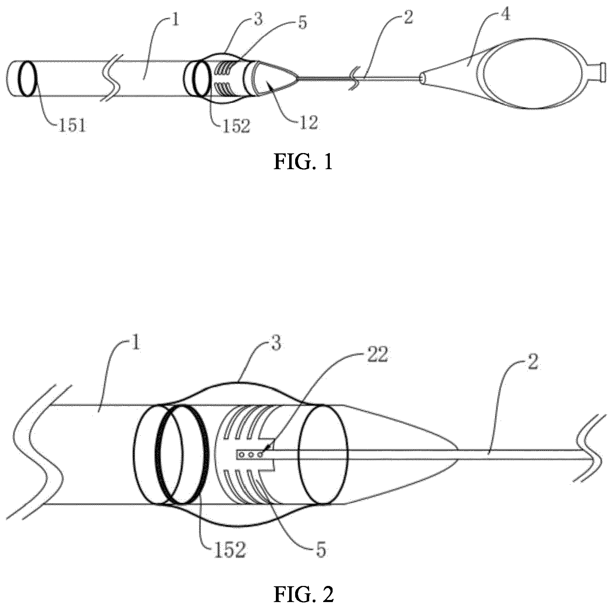

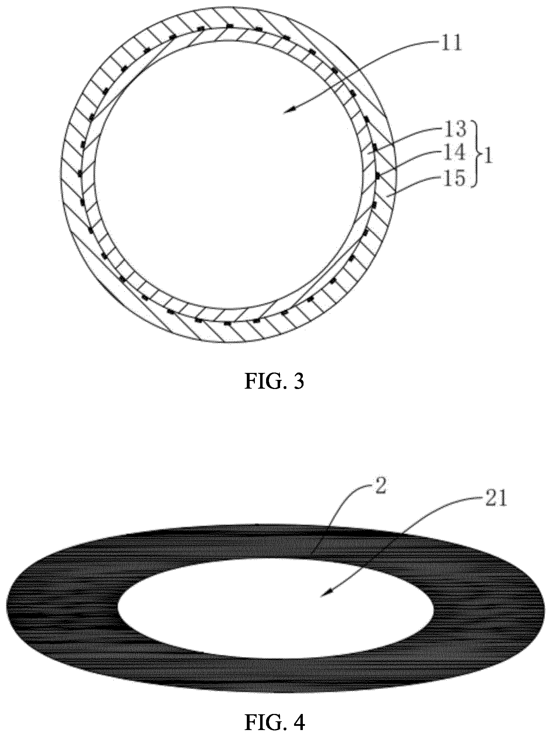

Referring to FIGs. 1, 3, and 4, an extension catheter provided by the embodiments of the present application is described. The extension catheter includes a catheter body 1, a push rod 2, and a balloon 3. The interior of the catheter body 1 is provided with an extension channel 11 for conveying a medical device, and a guiding port 12 is provided at the proximal end of the catheter body 1. The push rod 2 is connected to the proximal end of the catheter body 1 and forms a connecting portion. The interior of the push rod 2 is provided with an inflation channel 21. The balloon 3 is provided on the catheter body 1 at the proximal end and located at the connecting portion, and its interior is in communication with the inflation channel 21.

In the extension catheter of the present application, a balloon 3 is provided at the proximal end of the catheter body 1, and the inflation channel 21 that communicates with the balloon 3 is provided inside the push rod 2. The extension catheter is inserted along the proximal end of the guide catheter 6, with one end of the push rod 2 protruding from the proximal end of the guide catheter 6 and the distal end of the catheter body 1 protruding from the distal end of the guide catheter 6. The proximal end of the catheter body 1 is located at the distal end of the guide catheter 6. The balloon 3 can be inflated through the inflation channel 21. Since the inflation channel 21 is configured inside the push rod 2, thus the radial size of the push rod 2 is reduced, thereby facilitating the delivery of other devices. After expansion, the outer wall of the balloon 3 is tightly adhered to the inner cavity of the guide catheter 6, thereby preventing the extension catheter from slipping or dislodging within the guide catheter 6 and enhancing the axial stability of the extension catheter within the guide catheter 6.

In one embodiment, referring to FIGs. 1, 3, and 4, the catheter body 1 includes an inner layer 13, a reinforcement layer 14, and an outer layer 15, arranged from the inside to the outside. The extension channel 11 is located within the inner layer 13. Such a three-layer composite structure provides strong support, pressure resistance, and anti-kinking properties.

The material of the inner layer 13 is polytetrafluoroethylene (PTFE) or linear low-density polyethylene (LLDPE). PTFE has high lubricity and non-stick properties, which facilitates the passage of devices through the extension channel 11 within the inner layer 13. LLDPE has a higher softening and melting temperature, with advantages such as high strength, good toughness, rigidity, heat resistance, cold resistance, and excellent resistance to environmental stress cracking, impact strength, and tear strength.

The material of the outer layer 15 is selected from one or more of polyether-block-polyamide, nylon, and polyurethane elastomer, ensuring a smoother appearance and tactile feel on the surface of the catheter body 1. These materials can fully protect the blood vessels from thrombosis, dissection, and other complications.

The hardness of the material of the outer layer 15 decreases progressively from the proximal end to the distal end of the catheter. This not only prevents deformation but also facilitates passage through tortuous lesion sites. The use of flexible material at the distal end can avoid damage to the vessel walls during advancement, thereby better meeting the needs of catheter manipulation within human blood vessels. In such a way, it allows for more precise and convenient operation by physicians while reducing patient discomfort during surgery.

In one embodiment, referring to FIGs. 1, 3, and 4, the reinforcement layer 14 includes a stainless steel braid or a spring, which enhances the strong support, pressure resistance, and anti-kinking properties of the extension catheter.

In one embodiment, referring to FIGs. 1, 3, and 4, the balloon 3 is made of one of the following materials: silicone rubber, polyurethane elastomer, and thermoplastic elastomer. The silicone rubber is characterized by its physiological inertness and non-thrombogenic properties, which is widely used in the medical field therefore. The polyurethane resin, as a high-strength, tear-resistant, and wear-resistant polymer material, is widely used in daily life, industrial production, and medicine. The thermoplastic elastomers (TPE/TPR), also known as artificial or synthetic rubber, combine the excellent properties of traditional crosslinked rubber, such as high elasticity, aging resistance, and oil resistance, with the convenient processing characteristics of ordinary plastics.

In one embodiment, referring to FIGs. 1, 3, and 4, the cross-sectional shape of the push rod 2 is elliptical, circular, or semi-circular. An elliptical push rod or a semi-circular push rod allows for a thinner profile, saving space and facilitating the passage of other instruments. A circular push rod 2 ensures better strength and improved pushability.

In one embodiment, referring to FIGs. 1, 3, and 4, the material of the push rod 2 is hypotube. The hypotube has high rigidity and excellent anti-kinking properties, which obtains improved pushability.

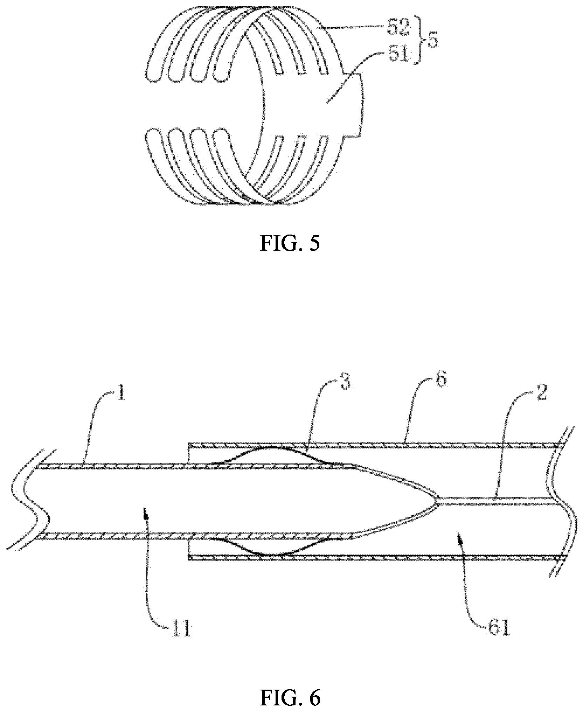

In one embodiment, referring to FIGs. 1, 2, 3, and 5, the extension catheter further includes a connecting member 5 that connects the reinforcement layer 14 and the push rod 2. The connecting member 5 is made of metal, and the connection between the connecting member 5, the push rod 2, and the reinforcement layer 14 is achieved through laser welding. In such a way, a firm connection between the push rod 2 and the catheter body 1 is ensured, with the connection point located in the middle of the catheter body 1 to avoid vascular injury.

In one embodiment, referring to FIGs. 1, 2, 3, and 5, the connecting member 5 includes a connecting plate 51 and multiple connecting strips 52 attached to the opposite sides of the connecting plate 51. The connecting plate 51 and each connecting strip 52 are connected to the reinforcement layer 14, with the push rod 2 connected to the connecting plate 51 and the connecting strips 52 wrapping around and connecting to the inner layer 13. The circumferential wrapping of the connecting strips 52 around the inner layer 13 ensures a stable connection of the connecting member 5 to the catheter body 1.

In one embodiment, referring to FIGs. 1 and 2, the distal end of the push rod 2 is provided with a first through-hole 22, which is communicated with the inflation channel 21. The outer layer 15 is provided with a second through-hole positioned to correspond with the first through-hole 22. The inflation channel 21 is communicated with the interior of the balloon 3 through the first and second through-holes. Multiple first through-holes 22 are arranged in parallel, which facilitates the injection of fluid or other media through the inflation channel 21 to inflate the balloon 3, thereby allowing the balloon 3 to quickly anchor and seal within the guide catheter 6.

In one embodiment, referring to FIGs. 1, 2, and 3, the guiding port 12 is beveled. The proximal end of the catheter body 1 is beveled, with the cross-sectional area of the beveled guiding port 12 being larger than that of the extension channel 11, which facilitates the smooth passage of other instruments through the guiding port 12 into the extension channel 11.

In one embodiment, referring to FIGs. 1, 2, 3, and 4, the extension catheter further includes a catheter hub 4 connected to the proximal end of the push rod 2. The catheter hub 4 is provided with a third through-hole that is communicated with the inflation channel 21. The presence of the catheter hub 4 at the proximal end of the push rod 2 can facilitate the manipulation by the operator.

In one embodiment, referring to FIGs. 1, 2, and 4, the distal end of the catheter body 1 is provided with a first radiopaque ring 151, and the proximal end of the catheter body 1 is provided with a second radiopaque ring 152 located inside the balloon 3. The arrangements of the first and second radiopaque rings 151 and 152 allow for precise positioning within the human body under fluoroscopy, thereby reducing the complexity of the procedure.

In one embodiment, referring to FIGs. 1, 2, and 3, the balloon 3 is vacuum-sealed and flatly adhered to the catheter body 1. The distal end of the balloon 3 is closely welded to the outer layer 15 of the catheter body 1 to form a sealed end, while the proximal end of the balloon 3 is closely welded to the outer layer 15 and the distal end of the push rod 2. The smooth transition at the welded ends of the balloon 3 can ensure minimal resistance and smooth passage for other instruments entering the channel.

Referring to FIGs. 1, 2, 3, and 6, another objective of the present application is to provide a medical device that includes a guide catheter 6 and an extension catheter. The guide catheter 6 is provided with a guide channel 61, and the proximal end of the catheter body 1 is equipped with a Y-shaped connector valve. The proximal end of the catheter body 1 is inserted into the guide channel 61, and the balloon 3, when inflated, is pressed against the inner wall of the guide channel 61.

In one embodiment, the medical device further includes an aspirator connected to the Y-shaped connector valve. The aspirator is communicated with the guide channel 61 and is used to aspirate thrombi.

The operation process of the medical device for thrombus and plaque aspiration using the external aspirator is as follows.

The guide catheter 6 is advanced to the coronary ostium. A guidewire is inserted through the Y-shaped connector valve at the proximal end of the guide catheter 6 to the distal end of the coronary stenosis. The extension catheter is then advanced along the guidewire, entering the guide catheter 6 through the Y-shaped connector valve. The distal end of the catheter body 1 protrude from the distal end of the guide catheter 6, while the proximal end of the catheter body 1 is located within the guide channel 61. One end of the push rod 2 is within the guide channel 61, and the other end protrudes from the Y-shaped connector valve, with the catheter hub 4 located outside the Y-shaped connector valve. Fluid is injected through the third through-hole in the catheter hub 4 and the inflation channel 21 within the push rod 2 to inflate the balloon 3. Once expanded, the balloon 3 is anchored within the guide channel 61, sealing the gap between the guide catheter 6 and the catheter body 1, and connecting the guide channel 61 with the extension channel 11. The interface of the push rod 2 protruding from the Y-shaped connector valve is sealed, and the aspirator is connected to the other interface of the Y-shaped connector valve, for aspirating the thrombi and plaques at the distal end of the catheter body 1. Due to the negative pressure, the thrombi or calcified plaques flow back through the extension channel 11 into the guide channel 61 until they are removed from the body.

The medical device of the present application utilizes the aforementioned extension catheter. When the balloon 3 positioned at the proximal end of the catheter body 1 and at distal end of the guide catheter 6 is expanded, the outer wall of the balloon 3 is tightly adhered to the inner cavity of the guide catheter 6, completely sealing the interior of the guide catheter 6. The balloon 3 is filled in the gap between the guide catheter 6 and the catheter body 1, thereby connecting the guide channel 61 with the extension channel 11. Once expanded, the balloon 3 prevents the extension catheter from slipping or dislodging within the guide catheter 6, thereby enhancing the axial stability of the extension catheter. The proximal end of the guide catheter 6 can be connected to an aspirator, which communicates with the extension channel 11 and the guide channel 61 to aspirate the thrombi and plaques. In such a way, it avoids the inconvenience of frequently replacing interventional catheters during surgery, reduces surgical time, lowers surgical complexity, and minimizes patient discomfort. Furthermore, the sealing effect of the balloon 3 reduces the likelihood of thrombus and plaque escape, thereby ensuring more thorough thrombus removal and reducing the risk of post-thrombotic syndrome.

The operation process of the medical device for delivering contrast agent/drug is as follows.

The guide catheter 6 is advanced to the coronary ostium. A guidewire is inserted through the Y-shaped connector valve at the proximal end of the guide catheter 6 to the distal end of the coronary stenosis. The extension catheter is then advanced along the guidewire, entering the guide catheter 6 through the Y-shaped connector valve. The distal end of the catheter body 1 protrude from the distal end of the guide catheter 6, while the proximal end of the catheter body 1 is located within the guide channel 61. One end of the push rod 2 is within the guide channel 61, and the other end protrudes from the Y-shaped connector valve, with the catheter hub 4 located outside the Y-shaped connector valve. Fluid is injected through the third through-hole in the catheter hub 4 and the inflation channel 21 within the push rod 2 to inflate the balloon 3. Once expanded, the balloon 3 is anchored within the guide channel 61, sealing the gap between the guide catheter 6 and the catheter body 1, and connecting the guide channel 61 with the extension channel 11. The interface of the push rod 2 protruding from the Y-shaped connector valve is sealed, and the contrast agent/drug is injected through the other interface of the Y-shaped connector valve. The contrast agent/drug flows through the guide channel 61 into the extension channel 11, exiting at the distal end of the catheter body 1 and flowing to the distal end of the coronary stenosis for superselective angiography/targeted drug delivery.

The medical device of the present application utilizes the aforementioned extension catheter. When the balloon 3 positioned at the proximal end of the catheter body 1 and at distal end of the guide catheter 6 is expanded, the outer wall of the balloon 3 is tightly adhered to the inner cavity of the guide catheter 6, completely sealing the interior of the guide catheter 6. The balloon 3 is filled the gap between the guide catheter 6 and the catheter body 1, thereby connecting the guide channel 61 with the extension channel 11. This allows the contrast agent/drug to flow from the guide channel 61 into the extension channel 11 for superselective angiography/targeted drug delivery. The superselective angiography can minimize the amount of contrast agent used, thereby reducing the risk of allergic reactions and minimizing X-ray exposure, making it highly practical. The targeted drug delivery can prevent the drug from entering non-target areas, thereby reducing side effects for the patient and improving drug utilization.

To verify the anchoring and sealing effectiveness of the medical device of the present embodiment, the inventors conducted a series of experiments. The compression ratio of the balloon 3 was used to indicate the sealing degree between the extension catheter and the guide catheter 6. The compression ratio is obtained by the cross-sectional area of the balloon 3 restricted within the guide catheter 6 dividing by the cross-sectional area of the balloon 3 not restricted by the guide catheter 6. A smaller compression ratio indicates a higher degree of compression and a greater ability to withstand aspiration negative pressure. The experimental data is shown in Table 1.

**Table 1: Test Data for Embodiments 1-9**

| Embodiment | Inflation Volume /mL | Balloon Length /mm | Balloon Diameter Change /mm | Pressure Tolerance of Guide Catheter /mmHg | Inner Diameter of Guide Catheter /mm | Compression Ratio of Balloon Volume |
|---|---|---|---|---|---|---|
| 1 | 0.5 | 8 | 0.10 | 550 | 2 | 60.00% |
| 2 | 1.0 | 8 | 0.18 | 800 | 2 | 77.78% |
| 3 | 1.5 | 8 | 0.24 | 1200 | 2 | 83.33% |
| 4 | 0.5 | 10 | 0.07 | 350 | 2 | 42.86% |
| 5 | 1.0 | 10 | 0.13 | 700 | 2 | 69.23% |
| 6 | 1.5 | 10 | 0.18 | 800 | 2 | 77.78% |
| 7 | 0.5 | 15 | 0.05 | 200 | 2 | 20.00% |
| 8 | 1.0 | 15 | 0.10 | 530 | 2 | 60.00% |
| 9 | 1.5 | 15 | 0.14 | 760 | 2 | 71.43% |

The test data in Table 1 shows that the extension catheters of Embodiments 1-9 can be used with the same guide catheter 6 and are suitable for various working conditions. The compression ratios range from 20% to 83%, which meets the requirements for sealing.

The above descriptions are merely preferred embodiments of the present application and are not intended to limit the scope of the application. Any modifications, equivalent substitutions, or improvements made within the spirit and principles of the present application should be included within the protection scope of the present application.

## Claims

1. An extension catheter, comprising:
a catheter body, an interior of the catheter body being provided with an extension channel for conveying a medical device, and a proximal end of the catheter body being provided with a guiding port;
a push rod, connected to the proximal end of the catheter body and forming a connecting portion, and an interior of the push rod being provided with an inflation channel; and
a balloon, provided on the catheter body and located at the proximal end of the catheter body, and an interior of the balloon being in communication with the inflation channel.

2. The extension catheter according to claim 1, wherein the catheter body comprises an inner layer, a reinforcement layer, and an outer layer arranged from inside to outside, and the extension channel is located at a center of the inner layer.

3. The extension catheter according to claim 2, further comprising a connecting member that is connected with the reinforcement layer and the push rod.

4. The extension catheter according to claim 3, wherein the connecting member comprises a connecting plate and multiple connecting strips connected to opposite sides of the connecting plate, the connecting plate and each of the connecting strips are connected to the reinforcement layer, the push rod is connected to the connecting plate, and each of the connecting strips wraps around and connects to the inner layer.

5. The extension catheter according to claim 3, wherein a distal end of the push rod is provided with a first through-hole in communication with the inflation channel, and the outer layer is provided with a second through-hole corresponding to a position of the first through-hole, and the inflation channel is in communication with the interior of the balloon through the first and second through-holes.

6. The extension catheter according to any one of claims 1 to 5, wherein the guiding port is arranged in a beveled manner.

7. The extension catheter according to any one of claims 1 to 5, further comprising a catheter hub connected to a proximal end of the push rod, the catheter hub being provided with a third through-hole in communication with the inflation channel.

8. The extension catheter according to any one of claims 1 to 5, wherein a distal end of the catheter body is provided with a first radiopaque ring, and the proximal end of the catheter body is provided with a second radiopaque ring located inside the balloon.

9. A medical device, comprising:
a guide catheter, an interior of the guide catheter being provided with a guide channel; and
the extension catheter according to any one of claims 1 to 8, the proximal end of the catheter body being inserted into the guide channel, and the balloon, when expanded, abutting against an inner wall of the guide channel.

10. The medical device according to claim 9, further comprising an aspirator connected to a proximal end of the guide catheter, the aspirator being in communication with the guide channel and used for aspirating thrombi.
